# EUROPEAN PATENT APPLICATION

(11) **EP 2 161 339 A1**
(43) Date of publication of application: **10.03.2010**
(21) Application number: 08163320.8
(22) Date of filing: 29.08.2008
(51) Int. Cl.: C12N 15/63, C12N 15/65, G01N 33/53

(54) **ADCC with modified NK92 cells**

(71) Applicant: F. Hoffmann-La Roche AG, 4070 Basel (CH); GlycArt Biotechnology AG, 8952 Schlieren (Zürich) (CH)
(72) Inventor: Grau, Roger, 8409 Winterthur (CH); Marti, Stefan, 4106 Therwil (CH); Schnueriger, Alfred, 4059 Basel (CH); Sondermann, Peter, 82131 Stockdorf (DE)
(74) Representative: Küng, Peter

(57) **Abstract**

The present invention relates to a method for producing FcγIIIa receptor expressing NK92 cell comprising a) providing a plasmid which comprises DNA encoding FcγIIIa receptor and a selection marker and b) introducing said plasmid with electroporation into NK92 cells Furthermore, the invention relates to an in vitro method for assessing the efficacy of an antibody to induce NK mediated ADCC comprising: a) producing an effector cell with the described producing method for modified NK92 cells, wherein said effector cell is a modified NK92 cell expressing FcγIIIa receptors, b)exposing a labeled target cell to an antibody of interest and the effector cell, c) monitoring the target cell for cytotoxicity, cytolysis or apoptosis with a nonradioactive readout-system.

SEQUENCE LISTING

<110>  F. Hoffmann-La Roche AG

<120>  ADCC with modified NK92 cells

<130>  25338

<160>  8

<170>  PatentIn version 3.4

<210>  1
<211>  794
<212>  DNA
<213>  Homo sapiens


<220>
<221>  CDS
<222>  (19)..(783)

<220>
<221>  allele
<222>  (544)..(544)
<223>  polymorphism T/G

<400>  1
cgccggccgg ccgccacc atg tgg cag ctg ctc ctc cca act gct ctg cta        51
                    Met Trp Gln Leu Leu Leu Pro Thr Ala Leu Leu
                    1               5                   10

ctt cta gtt tca gct ggc atg cgg act gaa gat ctc cca aag gct gtg        99
Leu Leu Val Ser Ala Gly Met Arg Thr Glu Asp Leu Pro Lys Ala Val
            15                  20                  25

gtg ttc ctg gag cct caa tgg tac agg gtg ctc gag aag gac agt gtg       147
Val Phe Leu Glu Pro Gln Trp Tyr Arg Val Leu Glu Lys Asp Ser Val
        30                  35                  40

act ctg aag tgc cag gga gcc tac tcc cct gag gac aat tcc aca cag       195
Thr Leu Lys Cys Gln Gly Ala Tyr Ser Pro Glu Asp Asn Ser Thr Gln
    45                  50                  55

tgg ttt cac aat gag agc ctc atc tca agc cag gcc tcg agc tac ttc       243
Trp Phe His Asn Glu Ser Leu Ile Ser Ser Gln Ala Ser Ser Tyr Phe
60                  65                  70                  75

att gac gct gcc aca gtc gac gac agt gga gag tac agg tgc cag aca       291
Ile Asp Ala Ala Thr Val Asp Asp Ser Gly Glu Tyr Arg Cys Gln Thr
                80                  85                  90

aac ctc tcc acc ctc agt gac ccg gtg cag cta gaa gtc cat atc ggc       339
Asn Leu Ser Thr Leu Ser Asp Pro Val Gln Leu Glu Val His Ile Gly
            95                  100                 105

tgg ctg ttg ctc cag gcc cct cgg tgg gtg ttc aag gag gaa gac cct       387
Trp Leu Leu Leu Gln Ala Pro Arg Trp Val Phe Lys Glu Glu Asp Pro
        110                 115                 120

att cac ctg agg tgt cac agc tgg aag aac act gct ctg cat aag gtc       435
Ile His Leu Arg Cys His Ser Trp Lys Asn Thr Ala Leu His Lys Val
    125                 130                 135

aca tat tta cag aat ggc aaa ggc agg aag tat ttt cat cat aat tct       483
Thr Tyr Leu Gln Asn Gly Lys Gly Arg Lys Tyr Phe His His Asn Ser
140                 145                 150                 155

gac gtc tac att cca aaa gcc aca ctc aaa gac agc ggc tcc tac ttc       531

Asp Val Tyr Ile Pro Lys Ala Thr Leu Lys Asp Ser Gly Ser Tyr Phe
                160                 165                 170

tgc agg ggg ctt ttt ggg agt aaa aat gtg tct tca gag act gtg aac 579
Cys Arg Gly Leu Phe Gly Ser Lys Asn Val Ser Ser Glu Thr Val Asn
            175                 180                 185

atc acc atc act caa ggt ttg gca gtg tca acc atc tca tca ttc ttt 627
Ile Thr Ile Thr Gln Gly Leu Ala Val Ser Thr Ile Ser Ser Phe Phe
        190                 195                 200

cca cct ggg tac caa gtc tct ttc tgc ttg gtg atg gta ctc ctt ttt 675
Pro Pro Gly Tyr Gln Val Ser Phe Cys Leu Val Met Val Leu Leu Phe
    205                 210                 215

gca gtg gac aca gga cta tat ttc tct gtg aag aca aac att cga agc 723
Ala Val Asp Thr Gly Leu Tyr Phe Ser Val Lys Thr Asn Ile Arg Ser
220                 225                 230                 235

tca aca aga gac tgg aag gac cat aaa ttt aaa tgg aga aag gac cct 771
Ser Thr Arg Asp Trp Lys Asp His Lys Phe Lys Trp Arg Lys Asp Pro
                240                 245                 250

caa gac aaa tga ggatctacgc g 794
Gln Asp Lys

<210> 2
<211> 254
<212> PRT
<213> Homo sapiens

<400> 2

Met Trp Gln Leu Leu Leu Pro Thr Ala Leu Leu Leu Leu Val Ser Ala
1               5                   10                  15

Gly Met Arg Thr Glu Asp Leu Pro Lys Ala Val Val Phe Leu Glu Pro
            20                  25                  30

Gln Trp Tyr Arg Val Leu Glu Lys Asp Ser Val Thr Leu Lys Cys Gln
        35                  40                  45

Gly Ala Tyr Ser Pro Glu Asp Asn Ser Thr Gln Trp Phe His Asn Glu
    50                  55                  60

Ser Leu Ile Ser Ser Gln Ala Ser Ser Tyr Phe Ile Asp Ala Ala Thr
65                  70                  75                  80

Val Asp Asp Ser Gly Glu Tyr Arg Cys Gln Thr Asn Leu Ser Thr Leu
                85                  90                  95

Ser Asp Pro Val Gln Leu Glu Val His Ile Gly Trp Leu Leu Leu Gln
            100                 105                 110

Ala Pro Arg Trp Val Phe Lys Glu Glu Asp Pro Ile His Leu Arg Cys
        115                 120                 125

His Ser Trp Lys Asn Thr Ala Leu His Lys Val Thr Tyr Leu Gln Asn
    130                 135                 140

Gly Lys Gly Arg Lys Tyr Phe His His Asn Ser Asp Val Tyr Ile Pro
145                 150                 155                 160

Lys Ala Thr Leu Lys Asp Ser Gly Ser Tyr Phe Cys Arg Gly Leu Phe
                165                 170                 175

Gly Ser Lys Asn Val Ser Ser Glu Thr Val Asn Ile Thr Ile Thr Gln
            180                 185                 190

Gly Leu Ala Val Ser Thr Ile Ser Ser Phe Phe Pro Pro Gly Tyr Gln
        195                 200                 205

Val Ser Phe Cys Leu Val Met Val Leu Leu Phe Ala Val Asp Thr Gly
    210                 215                 220

Leu Tyr Phe Ser Val Lys Thr Asn Ile Arg Ser Ser Thr Arg Asp Trp
225                 230                 235                 240

Lys Asp His Lys Phe Lys Trp Arg Lys Asp Pro Gln Asp Lys
                245                 250

<210>  3
<211>  254
<212>  PRT
<213>  Homo sapiens

<220>
<221>  SIGNAL
<222>  (1)..(18)

<400>  3

Met Trp Gln Leu Leu Leu Pro Thr Ala Leu Leu Leu Leu Val Ser Ala
1               5                   10                  15

Gly Met Arg Thr Glu Asp Leu Pro Lys Ala Val Val Phe Leu Glu Pro
            20                  25                  30

Gln Trp Tyr Arg Val Leu Glu Lys Asp Ser Val Thr Leu Lys Cys Gln
        35                  40                  45

Gly Ala Tyr Ser Pro Glu Asp Asn Ser Thr Gln Trp Phe His Asn Glu
    50                  55                  60

Ser Leu Ile Ser Ser Gln Ala Ser Ser Tyr Phe Ile Asp Ala Ala Thr
65                  70                  75                  80

Val Asp Asp Ser Gly Glu Tyr Arg Cys Gln Thr Asn Leu Ser Thr Leu
                85                  90                  95

Ser Asp Pro Val Gln Leu Glu Val His Ile Gly Trp Leu Leu Leu Gln
            100                 105                 110

Ala Pro Arg Trp Val Phe Lys Glu Glu Asp Pro Ile His Leu Arg Cys
        115                 120                 125

His Ser Trp Lys Asn Thr Ala Leu His Lys Val Thr Tyr Leu Gln Asn
    130                 135                 140

Gly Lys Gly Arg Lys Tyr Phe His His Asn Ser Asp Val Tyr Ile Pro
145                 150                 155                 160

Lys Ala Thr Leu Lys Asp Ser Gly Ser Tyr Phe Cys Arg Gly Leu Phe
                165                 170                 175

Gly Ser Lys Asn Val Ser Ser Glu Thr Val Asn Ile Thr Ile Thr Gln
            180                 185                 190

Gly Leu Ala Val Ser Thr Ile Ser Ser Phe Phe Pro Pro Gly Tyr Gln
        195                 200                 205

Val Ser Phe Cys Leu Val Met Val Leu Leu Phe Ala Val Asp Thr Gly
    210                 215                 220

Leu Tyr Phe Ser Val Lys Thr Asn Ile Arg Ser Ser Thr Arg Asp Trp
225                 230                 235                 240

Lys Asp His Lys Phe Lys Trp Arg Lys Asp Pro Gln Asp Lys
                245                 250

<210>  4
<211>  254
<212>  PRT
<213>  homo sapiens

<220>
<221>  SIGNAL
<222>  (1)..(18)

<400>  4

Met Trp Gln Leu Leu Leu Pro Thr Ala Leu Leu Leu Leu Val Ser Ala
1               5                   10                  15

Gly Met Arg Thr Glu Asp Leu Pro Lys Ala Val Val Phe Leu Glu Pro
            20                  25                  30

Gln Trp Tyr Arg Val Leu Glu Lys Asp Ser Val Thr Leu Lys Cys Gln
        35                  40                  45

Gly Ala Tyr Ser Pro Glu Asp Asn Ser Thr Gln Trp Phe His Asn Glu
    50                  55                  60

Ser Leu Ile Ser Ser Gln Ala Ser Ser Tyr Phe Ile Asp Ala Ala Thr
65                  70                  75                  80

Val Asp Asp Ser Gly Glu Tyr Arg Cys Gln Thr Asn Leu Ser Thr Leu
                85                  90                  95

Ser Asp Pro Val Gln Leu Glu Val His Ile Gly Trp Leu Leu Leu Gln
            100                 105                 110

Ala Pro Arg Trp Val Phe Lys Glu Glu Asp Pro Ile His Leu Arg Cys
        115                 120                 125

His Ser Trp Lys Asn Thr Ala Leu His Lys Val Thr Tyr Leu Gln Asn
    130                 135                 140

Gly Lys Gly Arg Lys Tyr Phe His His Asn Ser Asp Val Tyr Ile Pro
145                 150                 155                 160

Lys Ala Thr Leu Lys Asp Ser Gly Ser Tyr Phe Cys Arg Gly Leu Val
                165                 170                 175

Gly Ser Lys Asn Val Ser Ser Glu Thr Val Asn Ile Thr Ile Thr Gln
            180                 185                 190

Gly Leu Ala Val Ser Thr Ile Ser Ser Phe Phe Pro Pro Gly Tyr Gln
        195                 200                 205

Val Ser Phe Cys Leu Val Met Val Leu Leu Phe Ala Val Asp Thr Gly
    210                 215                 220

Leu Tyr Phe Ser Val Lys Thr Asn Ile Arg Ser Ser Thr Arg Asp Trp
225                 230                 235                 240

Lys Asp His Lys Phe Lys Trp Arg Lys Asp Pro Gln Asp Lys
                245                 250

<210>  5
<211>  300
<212>  DNA
<213>  homo sapiens

<220>
<221>  CDS
<222>  (6)..(266)

<400>  5
ccacc atg att cca gca gtg gtc ttg ctc tta ctc ctt ttg gtt gaa caa      50
      Met Ile Pro Ala Val Val Leu Leu Leu Leu Leu Leu Val Glu Gln
      1               5                   10                  15

gca gcg gcc ctg gga gag cct cag ctc tgc tat atc ctg gat gcc atc        98
Ala Ala Ala Leu Gly Glu Pro Gln Leu Cys Tyr Ile Leu Asp Ala Ile
                20                  25                  30

ctg ttt ctg tat gga att gtc ctc acc ctc ctc tac tgt cga ctg aag       146
Leu Phe Leu Tyr Gly Ile Val Leu Thr Leu Leu Tyr Cys Arg Leu Lys
            35                  40                  45

atc caa gtg cga aag gca gct ata acc agc tat gag aaa tca gat ggt       194
Ile Gln Val Arg Lys Ala Ala Ile Thr Ser Tyr Glu Lys Ser Asp Gly

        50                  55                  60

gtt tac acg ggc ctg agc acc agg aac cag gag act tac gag act ctg 242
Val Tyr Thr Gly Leu Ser Thr Arg Asn Gln Glu Thr Tyr Glu Thr Leu
    65                  70                  75

aag cat gag aaa cca cca cag tag ggatctacgc gtaataaaag atcattaatt 296
Lys His Glu Lys Pro Pro Gln
80                  85

tcat 300

<210> 6
<211> 86
<212> PRT
<213> homo sapiens

<400> 6

Met Ile Pro Ala Val Val Leu Leu Leu Leu Leu Leu Val Glu Gln Ala
1               5                   10                  15

Ala Ala Leu Gly Glu Pro Gln Leu Cys Tyr Ile Leu Asp Ala Ile Leu
            20                  25                  30

Phe Leu Tyr Gly Ile Val Leu Thr Leu Leu Tyr Cys Arg Leu Lys Ile
        35                  40                  45

Gln Val Arg Lys Ala Ala Ile Thr Ser Tyr Glu Lys Ser Asp Gly Val
    50                  55                  60

Tyr Thr Gly Leu Ser Thr Arg Asn Gln Glu Thr Tyr Glu Thr Leu Lys
65                  70                  75                  80

His Glu Lys Pro Pro Gln
                85

<210> 7
<211> 625
<212> DNA
<213> homo sapiens

<220>
<221> CDS
<222> (24)..(623)

<400> 7
ggcgcgccgg gtagccggct agc atg acc gag tac aag ccc acg gtg cgc ctc 53
Met Thr Glu Tyr Lys Pro Thr Val Arg Leu
1               5                   10

gcc acc cgc gac gac gtc ccc cgg gcc gta cgc acc ctc gcc gcc gcg 101
Ala Thr Arg Asp Asp Val Pro Arg Ala Val Arg Thr Leu Ala Ala Ala
                15                  20                  25

ttc gcc gac tac ccc gcc acg cgc cac acc gtc gac ccg gac cgc cac 149
Phe Ala Asp Tyr Pro Ala Thr Arg His Thr Val Asp Pro Asp Arg His
            30                  35                  40

atc gag cgg gtc acc gag ctg caa gaa ctc ttc ctc acg cgc gtc ggg 197
Ile Glu Arg Val Thr Glu Leu Gln Glu Leu Phe Leu Thr Arg Val Gly

45                      50                      55

ctc gac atc ggc aag gtg tgg gtc gcg gac gac ggc gcc gcg gtg gcg          245
Leu Asp Ile Gly Lys Val Trp Val Ala Asp Asp Gly Ala Ala Val Ala
    60                      65                      70

gtc tgg acc acg ccg gag agc gtc gaa gcg ggg gcg gtg ttc gcc gag          293
Val Trp Thr Thr Pro Glu Ser Val Glu Ala Gly Ala Val Phe Ala Glu
75                      80                      85                      90

atc ggc ccg cgc atg gcc gag ttg agc ggt tcc cgg ctg gcc gcg cag          341
Ile Gly Pro Arg Met Ala Glu Leu Ser Gly Ser Arg Leu Ala Ala Gln
                95                      100                     105

caa cag atg gaa ggc ctc ctg gcg ccg cac cgg ccc aag gag ccc gcg          389
Gln Gln Met Glu Gly Leu Leu Ala Pro His Arg Pro Lys Glu Pro Ala
            110                     115                     120

tgg ttc ctg gcc acc gtc ggc gtc tcg ccc gac cac cag ggc aag ggt          437
Trp Phe Leu Ala Thr Val Gly Val Ser Pro Asp His Gln Gly Lys Gly
        125                     130                     135

ctg ggc agc gcc gtc gtg ctc ccc gga gtg gag gcg gcc gag cgc gcc          485
Leu Gly Ser Ala Val Val Leu Pro Gly Val Glu Ala Ala Glu Arg Ala
    140                     145                     150

ggg gtg ccc gcc ttc ctg gag acc tcc gcg ccc cgc aac ctc ccc ttc          533
Gly Val Pro Ala Phe Leu Glu Thr Ser Ala Pro Arg Asn Leu Pro Phe
155                     160                     165                     170

tac gag cgg ctc ggc ttc acc gtc acc gcc gac gtc gag gtg ccc gaa          581
Tyr Glu Arg Leu Gly Phe Thr Val Thr Ala Asp Val Glu Val Pro Glu
                175                     180                     185

gga ccg cgc acc tgg tgc atg acc cgc aag ccc ggt gcc tga gc               625
Gly Pro Arg Thr Trp Cys Met Thr Arg Lys Pro Gly Ala
            190                     195

<210>  8
<211>  199
<212>  PRT
<213>  homo sapiens

<400>  8

Met Thr Glu Tyr Lys Pro Thr Val Arg Leu Ala Thr Arg Asp Asp Val
1               5                   10                  15

Pro Arg Ala Val Arg Thr Leu Ala Ala Ala Phe Ala Asp Tyr Pro Ala
            20                  25                  30

Thr Arg His Thr Val Asp Pro Asp Arg His Ile Glu Arg Val Thr Glu
        35                  40                  45

Leu Gln Glu Leu Phe Leu Thr Arg Val Gly Leu Asp Ile Gly Lys Val
    50                  55                  60

Trp Val Ala Asp Asp Gly Ala Ala Val Ala Val Trp Thr Thr Pro Glu
65                  70                  75                  80

Ser Val Glu Ala Gly Ala Val Phe Ala Glu Ile Gly Pro Arg Met Ala
                85                  90                  95

Glu Leu Ser Gly Ser Arg Leu Ala Ala Gln Gln Gln Met Glu Gly Leu
            100                 105                 110

Leu Ala Pro His Arg Pro Lys Glu Pro Ala Trp Phe Leu Ala Thr Val
        115                 120                 125

Gly Val Ser Pro Asp His Gln Gly Lys Gly Leu Gly Ser Ala Val Val
    130                 135                 140

Leu Pro Gly Val Glu Ala Ala Glu Arg Ala Gly Val Pro Ala Phe Leu
145                 150                 155                 160

Glu Thr Ser Ala Pro Arg Asn Leu Pro Phe Tyr Glu Arg Leu Gly Phe
                165                 170                 175

Thr Val Thr Ala Asp Val Glu Val Pro Glu Gly Pro Arg Thr Trp Cys
            180                 185                 190

Met Thr Arg Lys Pro Gly Ala
        195

## Description

The clinical efficacy of a number of therapeutic antibodies such as e.g. MabThera® and Herceptin® depends at least in part on their ability to induce Antibody-Dependent Cell-mediated Cytotoxicity (ADCC). ADCC describes a mechanism, by which antibody-coated target cells are killed by Fc Receptor expressing effector cells. A substantial contribution of ADCC is mediated by natural killer (NK) cells. In vitro assays assessing ADCC activity ofNK cells are therefore valuable tools for the characterization of therapeutic antibodies.

ADCC involves activation ofNK cells and is dependent on the recognition of antibody-coated infected cells by Fc receptors on the surface of the NK cell. The Fc receptors recognize the Fc (crystalline) portion of antibodies such as IgG, which bind to the surface of a target cell. The most common Fc receptor that exists on the surface of NK Cell is called CD16 or FcγRIII. NK cell mediated ADCC is triggered, when antibody of the IgG1 or IgG3 subtype bound to the surface of a target cell interacts with e.g. an FcγRIII expressed by NK cells. Clustering of FcγRIII induces a signaling cascade in the NK cell resulting in the secretion of cytokines such as IFN-γ, and cytotoxic granules containing perforin and granzymes that enter the target cell and promote cell death by triggering apoptosis.

NK cells for ADCC assays are typically primary cells isolated from donated blood. NK cell lines such as NK-92 described in Gong et al, (Leukemia, 19948: 652-8) do not express endogenously FcγRIIIa and are therefore not suitable for ADCC assays.

Therefore, the present invention provides a method for producing a NK92 cell expressing FcγIIIa receptor comprising
a) providing a plasmid which comprises DNA encoding a FcγIIIa receptor and a selection marker, and
b) introducing said plasmid with electroporation into NK92 cells.

Preferably, the electroporated NK92 cells of step b) are subjecting to a selection scheme comprising :
1) transferring the electroporated NK92 cells after not less than 24 hours after the electroporation to a selection medium,
2) maintaining the electroporated NK92 cells for 2 to 6 weeks in said selection medium, more preferably, 2 to 4 weeks,.

Preferably, the electroporated NK92 cells are subjected to a limiting dilution while maintained in the selection medium.

The term "limiting dilution" refers to a dilution scheme wherein a solution (e.g. cell medium) comprising cells is diluted so that there is statistically ≤ 1 cell per well or tube.

The present invention also provides a NK92 cell line expressing transgenic FcγIIIa receptors produced by the above described method.

Preferably, the FcγIIIa Receptor is FcγIIIa 158V or FcγIIIa 158F.

In a preferred embodiment, the NK92 cells or the NK92 expressing exogenous FcγIIIa receptor are co-transfected with exogenous Interleukin-2.

The FcγIIIa receptor is a subtype of the FcγIII receptor. The gene encoding the FcγIIIa receptor comprises at position 559 a polymorphism (T/G, corresponds to position 544 in SEQ. ID NO: 1) which causes in the protein at position 158 (position 176 in SEQ. ID NO: 2 to 4, protein with an 18 amino acid signal sequence) a polymorphism which influences the binding affinity of the receptor to the Fc portion of the antibody (Wu et al, J. Clin. Invest. 1997, 100(5): 1059-1070). An FcγIIIa receptor with a Valine at position 158 (158V) (corresponds to position 176 in SEQ. ID 2 and 3) has a high binding affinity and with a Phenylalanine at position 158 (corresponds to position 176 in SEQ. ID NO: 4) the receptor has a low binding affinity to the Fc portion of the antibody. The polymorphism V158/F158 can co-occur with other polymorphisms in said receptor.

The term "electroporation" refers to an electrical treatment of cells that induces transient pores, through which DNA can enter the cell. Electroporation is a well known transfection method.

The term "selection marker" refers to a gene encoding for a protein which allows to select the successfully transfected cells after the transfection. In bacterial cells, for example beta-lactamase (bla gene) can be used. In mammalian cells, for example puromycin acetyltransferase (pac gene) can be used as selection marker.

The term "selection medium" refers to a medium which allows only those cells to grow which were successfully transfected. The selection is performed either by absence of a substance required for the growth or survival of said cell or a selection agent which negatively effects the untransformed cells. Alternatively, the selection agent is a substance required for the growth or survival of the cell, which can only be metabolized by the successfully transfected cells. As selection agent for bacterial cells, e.g. an antibiotic can be used.

In addition, the present invention provides an in vitro method for assessing the efficacy of an antibody to induce NK cell mediated ADCC comprising :
a) producing an effector cell by stably introducing the coding region for a FcγIIIa receptor into an NK92 cell using electroporation wherein said transfected NK92 cell expresses exogenous FcγIIIa receptors,
b)exposing a labeled target cell to an antibody of interest and the effector cell, and
c) monitoring the target cell for cytotoxicity, cytolysis or apoptosis with a non-radioactive readout-system.

The term "effector cell" as used herein refers to a cell that performs the damage of target cells membrane integrity and ultimately the cell death in response to the binding of effector cell receptors (i.e. FcγIII receptors) to the antibodies on the target cell or to cells that release cytotoxic compounds or signaling substances (e.g. cytokines) in response to antibody-Fc receptor binding (i.e. antibody-FcγIII receptor binding). In particular, an effector cell is a NK92 cell which expresses exogenous FcγIIIa receptor.

Preferably, the FcγIIIa Receptor expressing NK92-cell line used in the assay is a NK92 cell line expressing FcγIIIa 158V and/or a NK92 cell line expressing FcγIIIa 158F. More preferably, the FcγIIIa Receptor expressing NK92-cell line is produced by the above described producing method of modified NK92 cells.

Preferably, the selection scheme after the electroporation is as follows:
1) selection (e.g. with puromycin acetyltransferase) for positive transfection is done not earlier than 24 hours after the electroporation,
2) maintaining the transfected cells for 2 to 6 weeks in selection medium, more preferably, 2 to 4 weeks.

The term "antibody" or "antibody of interest" refers to antibodies comprising two heavy and two light chains. The antibody can be a monoclonal or polyclonal antibody, a human antibody, or a chimeric (preferably humanized) antibody. The monoclonal antibody can be mono-specific or bi-specific. A bi-specific antibody has a binding specificity for at least two different antigens or antigen epitopes. Preferably, the antibody of interest is a therapeutic antibody. A therapeutic antibody is a monoclonal antibody, often human or humanized, which is intended to treat a disease or disorder.

The term "cytotoxicity" refers to the property of killing cells.

Apoptosis or programmed cell death is a genetically determined process of cell self-destruction, that is activated either by the presence of a stimulus or by the removal of a stimulus or suppressing agent.

The term "cytolysis" refers to the destruction of a cell by rupturing of the cell membrane.

A "target cell" can be any cell, including a cell of a cell line and a cell isolated from an organism, organ, or tissue. The target cell can also be a cell comprising a exogenous gene. Preferably, the cell is a mammalian cell. More preferably, the cell is a human cell.

Optionally the target cell is loaded with a non-radioactive label before exposing the labeled target cell to an antibody of interest and an effector cell (step a)). Preferred non-radioactive labels are BADTA and Calcein Acetoxymethyl.

Non-radioactive readout systems are well known to the skilled person in the art. Suitable methods are described in the following.

### 1) TDA release

The non-radioactive label is BADTA, the acetocymethyl ester of the fluorescence enhancing ligand TDA. BADTA is membrane-permeable. Within the cells the ester bonds are hydrolyzed to form a hydrophilic ligand (TDA) which no longer passes the membrane. An ADCC-induced loss of the target cell's membrane integrity releases TDA into the supernatant. In contact with Europium Solution, TDA and Europium form a highly fluorescent and stable chelate. The measured signal correlates directly with the amount of lysed cells.

### 2) Calcein Release/Retention

The target cell is loaded with membrane-permeable Calcein Acetoxymethyl (Calcein-AM). Within the cells Calcein-AM is modified by endogenous esterases to Calcein which no longer passes the membrane. When effector cells are placed in contact with the loaded target cells, the latter lysed and cytoplasmic Calcein is released into the supernatant. Calcein is a fluophore and the measured signal (e.g. with MTP reader) correlates directly with the amount of lysed cells. Alternatively, remaining Calcein (retained Calcein) in target cells with intact cell membranes correlates reciprocally to the amount of lysed cells. To measure the amount of intact cells, cell containing fluids are pelletized by centrifugation, the supernatant is decanted and cell pellet containing the Calcein labeled target cells is lysed by Borate acid solution. The free Calcein is released into the supernatant and the measured signal (e.g. with MTP reader) correlates directly with the amount of the former intact cells.

### 3) Lactate Dehydrogenase (LHD) release

Lactate Dehydrogenase (LDH) is an intracellular enzyme that oxidizes lactate in the presence of NADH or reduces pyruvate in the presence of NAD. An ADCC-induced loss of the membrane integrity of the target cell releases endogenous cytoplasmic LDH into the supernatant. LDH in the supernatant is detectable via colorimetric reaction. The measured signal correlates directly with the amount of lysed cells.

### 4) De-granulation

Cross linking of Fc-receptors on NK-92 cells with antibodies results in an NK-cell intrinsic signaling pathway that leads to the release of the content of granula into the supernatant ('de-granulation'). The content of these granula partly consists in serine-esterases ('granzymes'). These esterases can be detected by densitometry after conversion of Nα-benzyloxycarbonyl-L-lysine thiobenzyl ester (BLT) substrate to an optically dense metabolite.

Alternatively, the granula may be detected with a by a monoclonal antibody directed against a granula specific marker. Said marker is the LAMP-1 peptide (CD107a) located on the inner surface of the granula membrane. When the granula have fused with the outer cell membrane, said CD107a marker is located on the extracellular side of the cell membrane and therefore easily detectable by an antibody. The anti-CD107a antibody can be visualized in a quantitative manner by flow cytometry. The de-granulation of NK-92 cells can be evoked either by cross-linking of Fc-receptors (like in the example above) or by initiating an immunological synapse with a target cell (like in an ADCC assay).

Preferred readout-systems are measuring of the TDA release or the Calcein retention. Preferably, the target cell is monitored for cytotoxicity, cytolysis, or apoptosis by determining the amount of released TDA or retained Calcein.

NK92 cells lack endogenous Interleukin-2 (IL-2). Therefore, it is preferred that exogenous DNA encoding IL-2 is incorporated into the FcγIII Receptor expressing NK92-cell line so that the modified NK92 co-expresses transgenic endogenous IL-2. Alternatively, IL-2 can be added to the cell medium (e.g. growth or selection medium).

The described FcγIIIa Receptor expressing NK92-cell line can be utilized for a series of other assays monitoring the functionality of therapeutic antibodies, for example for assessing the efficacy of an antibody to induce NK-mediated ADCC independent of a target cell. This can be done by measuring the release of cytotoxic compounds or signaling substances (e.g. cytokines) by said modified NK92-cells or by detecting Fc-receptor binding of antibodies on the modified NK-92 cell lines. Fc-receptor-binding is visualized by binding of a secondary antibody reagent to the analyte. This secondary antibody reagent is directly labeled either with fluorochromes for detection in fluorescence readers or by flow cytometry or is directly labeled with enzymes allowing densitometry detection of substrate conversion.

A further application is the use of the FcγIIIa Receptor expressing NK92-cell line in a proliferation assay (Proliferation of NK-92 cells upon stimulation with IL-2, IL-12, IL-15, IL-18 or IL-21 NK-cell proliferation-inducing cytokines or antibodies or antibody derivates mimicking the effects of NK-92 proliferation inducing cytokines and chemokines).

Furthermore, Fc Induction of target cell intrinsic autonomous death can be triggered upon cross-linking of molecules exposed to the cell surface by means of antibodies bound to by Fc-receptors on NK-cells. This functionality of Fc-receptor expressing NK-cells may be accomplished by cross-linking of the bound antibodies by secondary reagents and is independent ofNK-cell mediated effector functions. This can be secured by introducing mutations into the Fc-part of IgG1-like antibodies or usage of antibodies of the IgG4 type. NK-92 cells serve as cross-linking agent with -in this case- no active cytotoxic functionality and thus represent the in vivo situation.

Having now generally described this invention, the same will become better understood by reference to the specific examples, which are included herein for purpose of illustration only and are not intended to be limiting unless otherwise specified, in connection with the following figures.

### Figures

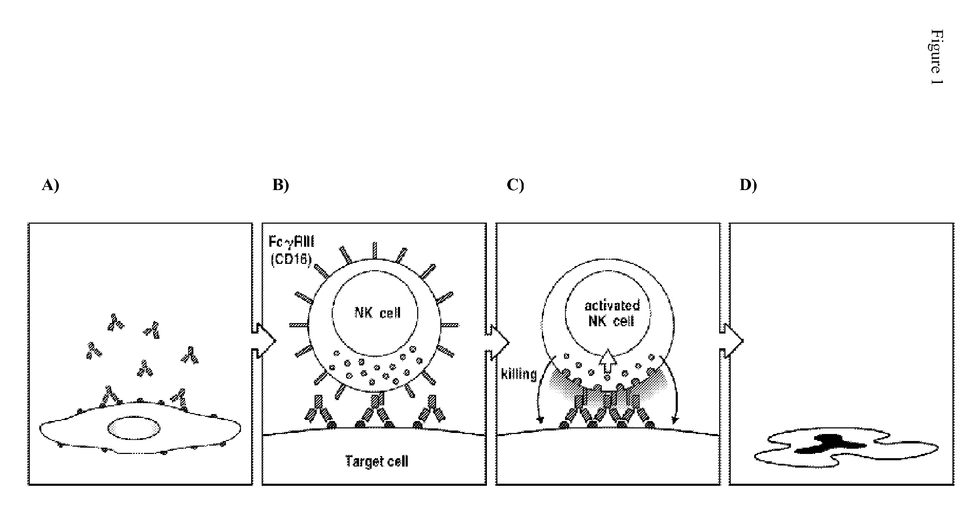

Figure 1 shows a schematic representation of an Antibody-Dependent Cell-mediated Cytotoxicity (ADCC) assay. A) The antibody binds antigens on the surface of the target cells. B) Fc receptors on effector cells recognize the bound antibodies. C) Cross-linking of Fc receptors signals the effector cell to kill the target cell. D) The target cell dies (see e.g. Charles A. Janeway et al., Immunobiology: the immune system in health and disease, 6^{th} ed.).

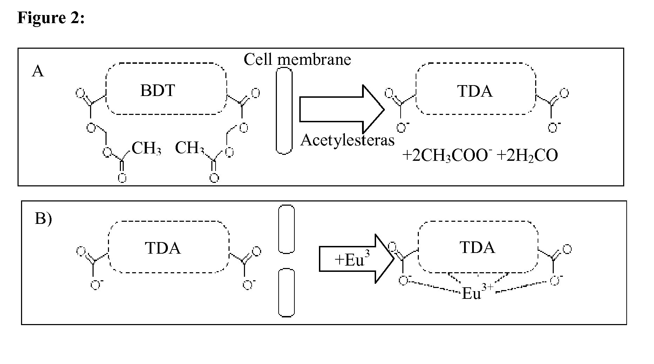

Figure 2 shows a schematic representation of a TDA release. A) BATDA is membrane permeable and within the cell BADTA is modified by endogenous esterases to TDA which no longer passes the membrane. B) After ADCC-induced damage of the membrane of the target cell, TDA is released into the supernatant. With Europium the released TDA form a stable fluorescent chelate.

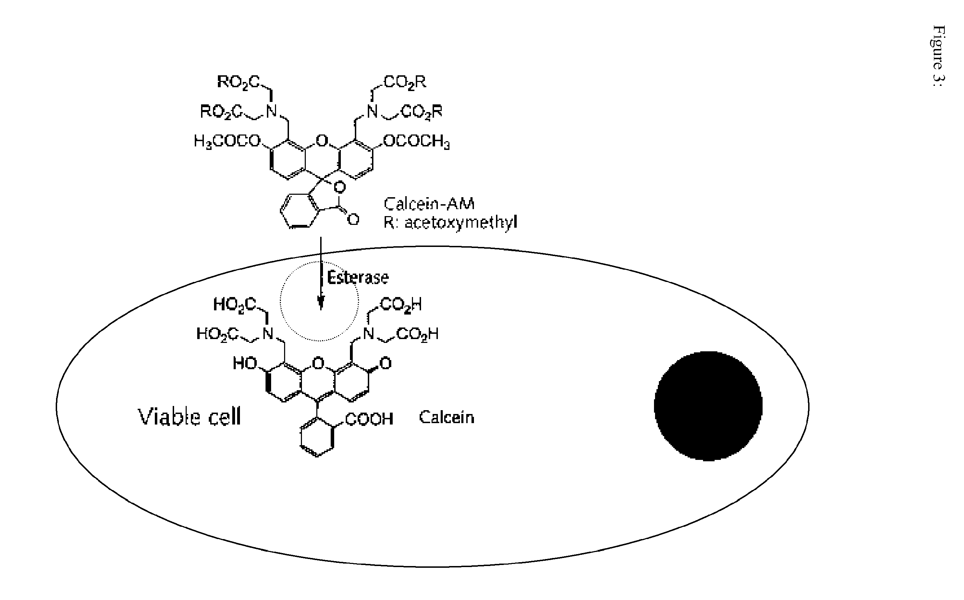

Figure 3 shows a schematic representation of a Calcein release. Calcein Acetoxymethyl (AM) is membrane permeable. Within the cell Calcein-AM is modified by endogenous esterases to Calcein, which no longer passes the membrane. After ADCC-induced damage of the membrane of the target cell, fluorescent Calcein is released into the supernatant.

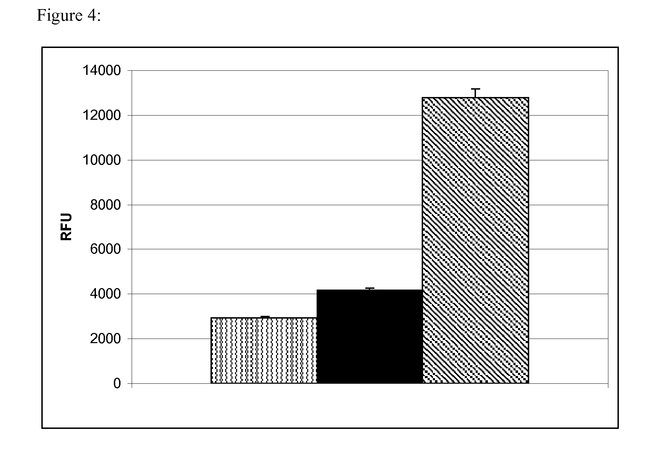

Figure 4 shows a graphical representation of the induced cytotoxicity of the antibody Rituximab with a FcγIIIa Receptor expressing NK92-cell line (NK92_6FD5). Target cells are WIL2-S cells (ATCC Cat# CRL-8885). ▩ Target cells, ■ Target cells + effector cells, ▧ Target cells + effector cells + Antibody.

### Examples:

Commercially available reagents referred to in the examples were used according to manufacturer's instructions unless otherwise indicated.

### Example 1: NK92- FcγRIIIa-F158 cells

NK-92 cells (ATCC CRL-2407) were grown in supplemented Minimal Essential Medium (MEM) Alpha Medium (Invitrogen, Carlsbad/CA, USA, containing Earle's Salts, L-glutamine but no Ribonucleosides or Deoxyribonucleosides), 10% (v/v) Fetal Bovine Serum (Invitrogen), 10% (v/v) horse serum (Invitrogen), 0.1mM 2-Mercaptoethanol, 0.2mM myo-Inositol (Sigma-Aldrich, St. Louis/MO, USA) 0.02mM Folic Acid (Sigma-Aldrich), 150U hIL-2/ml (Peprotech, Rocky Hill/NJ, USA). For transfection exponentially growing cells were harvested, washed once in non-supplemented MEMalpha and adjusted to a concentration of 3x10⁷ cells/ml in the same medium.

The plasmid used for transfection consisted of a pUC-vector (Vieira J, Messing J. 1982. Gene. 1982 Oct;19(3):259-68) containing the origin of replication and the *bla* gene for amplification and selection in E.coli modified by the insertion of the genes for human FcγRIIIa-F158 (SEQ ID NO: 1, with a T at position 544 of SEQ. ID NO: 1), FcRγ chain (SEQ ID NO: 5, amino acid sequence: SEQ. ID NO: 6) and Puromycin Acetyltransferase (pac gene, SEQ. ID NO: 7, amino acid sequence: SEQ. ID NO: 6). Each of the genes was under the control of a MPSV (Myeloproliferative sarcoma virus) promoter (Ferrara et al. 2006. Biotechnol Bioeng. 93(5):851-61).

400µl of the cell suspension was electroporated with 5µg of the linearized plasmid-DNA in a 4mm cuvette using a Gene Pulser (Bio-Rad Laboratories, Hercules/CA, USA) equipped with a 960µF capacitor. Transfection was performed with one exponential decay pulse at a voltage set to 220V and an unlimited resistance at 25°C. After transfection cells were transferred to complete MEMalpha and maintained at 37°C and 5% CO₂. 24h after transfection the medium was adjusted to 2µg/ml Puromycin (Sigma). Surviving cells were expanded and subcloned by limiting dilution. Expression of CD 16 was tested by staining with the FITC-labeled anti-FcγRIII antibody 3G8 (BD Bisciences, San Jose/CA, USA) and subsequent flowcytometric analysis. Positive, stable clones were further expanded and frozen in 90% (v/v) culture medium and 10% (v/v) DMSO (Hybri-Max, Sigma-Aldrich) at a cell density of 10⁷ cells/ml.

A NK-92 cell line expressing FcγRIIIa-V158 (SEQ ID NO: 1, with a G at position 544 of SEQ. ID NO: 1) was generated accordingly.

### Example 2: ADCC Assay

### Target cell labeling

WIL2-S cells (ATCC Cat# CRL-8885) were cultivated in RPMI1640 supplemented with 10% foetal calf serum, 20mM Hepes, 2mM L-Glutamine. Cell titer was adjusted to 1x10⁶ cells/ml. 2µl DELFIA BATDA ligand (Perkin Elmer Cat# C136-100) were added per 1ml cell suspension and cells were incubated for approximately 25 minutes in a humidified CO₂-incubator at 37°C. Labeled cells were centrifuged at 250xg for 3 minutes and the pellet was washed 3 times in PBS supplied with 5 mM sulfinpyrazone. The washed cells were suspended in WIL2-S growth medium and the cell density was adjusted to 0.2x10⁶ cells/ml.

### Effector cell preparation

NK92- FcγRIIIa-F158 cells (Glycart) were cultivated in MEM-alpha medium supplemented with 10% foetal calf serum, 10% horse serum, 0.1mM 2-mercaptoethanol, 0.2mM myo-inositol, 0.02mM folic acid, 5µg/ml puromycin and 10ng/ml IL-2. For assay purposes, NK92- FcγRIIIa-F158 cells were centrifuged at 250xg for 3 minutes and the cell pellet was resuspended in WIL2-S growth medium (RPMI1640 supplemented with 10% foetal calf serum, 20mM Hepes, 2mM L-Glutamine) at a density of 0.6x10⁶ cells/ml.

### Assay setup

Equal volumes of labeled target cells at 0.2x10⁶ cells/ml and effector cell suspension at 0.6x10⁶ cells/ml were carefully mixed and 100µl per well were transferred to a round-bottomed cell culture plate. Rituximab was diluted in WIL2-S growth medium (RPMI1640 supplemented with 10% foetal calf serum, 20mM Hepes, 2mM L-Glutamine) and 100µl per well were added to the target- effector cell mixture resulting in Rituximab assay concentrations ranging from 3pg/ml to 300 ng/ml. The assay plate was incubated in a humidified CO₂-incubator at 37°C for 2h. After incubation, the plate was centrifuged at 500xg for 5 minutes. 20µl of the supernatant from each well were transferred to a white 96 well plate. 200µl europium solution (Perkin Elmer Cat# C135-100) were added to each well, followed by a 15 minutes incubation on a plate shaker. Time-resolved fluorescence in RFU (relative fluorescence units) was measured using following settings: delay: 50 µs; integration: 1000 µs; excitation 345 nm; emission 615 nm; cut off 590 nm.

## Claims

**1.** A method for producing FcγIIIa receptor expressing NK92 cell comprising
a) providing a plasmid which comprises DNA encoding FcγIIIa receptor and a selection marker, and
b) introducing said plasmid with electroporation into NK92 cells.

**2.** A method according to claim 1 wherein the electroporated NK92 cells of step b) are subjecting to a selection scheme comprising :
1) transferring the electroporated NK92 cells after not less than 24hours after the electroporation to a selection medium,
2) maintaining the electroporated NK92 cell for 2 to 6 weeks in said selection medium.

**3.** A method according to claim 1 or 2, wherein the electroporated NK92 cells are subjected to a limiting dilution while maintained in said selection medium.

**4.** The method according to any one of claims 1 to 3, wherein the NK92 cells are co-transfected with exogenous Interleukin-2.

**5.** An in vitro method for assessing the efficacy of an antibody to induce NK mediated ADCC comprising:
a) producing an effector cell according to the method of any one of claims 1 to 4, wherein said effector cell is a NK92 cell expressing exogenous FcγIIIa receptor,
b) exposing a labeled target cell to an antibody of interest and the effector cell, and
c) monitoring the target cell for cytotoxicity, cytolysis or apoptosis with a non-radioactive readout-system.

**6.** The method according to claim 5, wherein the FcγIII Receptor expressing NK92-cell line is a FcγIIIa 158V expressing NK92 cell line and/or a FcγIIIa 158F expressing NK92 cell line.

**7.** The method according to any one of claims 5 to 6, wherein the target cell is loaded with a non-radioactive label before exposing the labeled target cell to an antibody of interest and an effector cell.

**8.** The method according to claim 7, wherein the non-radioactive label is BADTA, or Calcein Acetoxymethyl.

**9.** The method according to any one of claims 5 to 8, wherein the target cell is monitored for cytotoxicity, cytolysis or apoptosis by determining the amount of released TDA.

**10.** The method according to any one of claims 5 to 9, wherein the effector cell co-expresses transgenic Interleukin-2.

**12.** Methods and uses substantially as herein before described especially with reference to the foregoing examples.
